# EUROPEAN PATENT APPLICATION

(11) **EP 4 056 705 A1**
(43) Date of publication of application: **14.09.2022**
(21) Application number: 20887900.7
(22) Date of filing: 02.07.2020
(51) Int. Cl.: C12N 15/63, C12N 15/75, C12N 9/10, C12Q 1/6806

(54) **USE OF CAS9 PROTEIN FROM THE BACTERIUM PASTEURELLA PNEUMOTROPICA**

(30) Priority: 11.11.2019 RU 2019136164
(71) Applicant: Joint Stock Company "Biocad", 198515 Saint Petersburg (RU)
(72) Inventor: SEVERINOV, Konstantin Viktorovich, Moscow, 188301 (RU); SHMAKOV, Sergey Anatolievich, Voskresensk, 140200 (RU); ARTAMONOVA, Daria Nikolaevna, Moscow, 121359 (RU); GORYANIN, Ignaty Igorevich, Moscow, 117587 (RU); MUSHAROVA, Olga Sergeevna, Moscow, 123098 (RU); ANDREEVA, Julia Valerevna, Moscow, 142784 (RU); ZYUBKO, Tatiana Igorevna, Sankt-Peterburg, 197374 (RU); FEDOROVA, Iana Vitalevna, Gatchina, 188301 (RU); KHODORKOVSKII, Mikhail Alekseevich, Sankt- Peterburg, 195426 (RU); POBEGALOV, George Evgenevich, Sankt- Peterburg, 198205 (RU); ARSENIEV, Anatoliy Nikolaevich, Sankt- Peterburg, 195112 (RU); SELKOVA, Polina Anatolevna, Votkinsk, 427439 (RU); VASILIEVA, Aleksandra Andreevna, Sankt- Peterburg, 198334 (RU); ARTAMONOVA, Tatiana Olegovna, Sankt-Peterburg, 199406 (RU); ABRAMOVA, Marina Viktorovna, Sankt-Peterburg, 194021 (RU)
(74) Representative: Held, Stephan
(86) International application number: PCT/RU2020/050145
(87) International publication number: WO 2021/096391

(57) **Abstract**

The present invention describes a novel bacterial nuclease of the CRISPR-Cas9 system from the bacterium *P. pneumotropica*, as well as the use thereof to form strictly specific double-strand breaks in a DNA molecule. This nuclease has unusual properties and may be used as a tool for modifying the genomic DNA sequence in the cell of a unicellular organism or a multicellular organism. Thus, the versatility of the available CRISPR-Cas9 systems is increased, which fact will enable the use of various variants of Cas9 nucleases for cutting genomic or plasmid DNA in various organisms, in a larger number of specific sites and/or under various conditions.

## Description

### Field of the invention

The invention relates to biotechnology, specifically to novel enzymes, Cas nucleases of CRISPR-Cas systems, used for cutting DNA and editing the genome of various organisms. This technique may be used in the future for gene therapy of hereditary human diseases, as well as for editing the genome of other organisms.

### Background of the invention

DNA sequence modification is one of the topical problems in today's biotechnology field. Editing and modifying the genomes of eukaryotic and prokaryotic organisms, as well as manipulating DNA in vitro, require targeted introduction of double-strand breaks in DNA sequences.

To solve this problem, the following techniques are currently used: artificial nuclease systems containing domains of the zinc finger type, TALEN systems, and bacterial CRISPR-Cas systems. The first two techniques require laborious optimization of the nuclease amino acid sequence for recognition of a specific DNA sequence. In contrast, when it comes to CRISPR-Cas systems, the structures that recognize a DNA target are not proteins, but short guide RNAs. Cutting of a particular DNA target does not require the synthesis of nuclease or its gene de novo but is made by way of using guide RNAs complementary to the target sequence. It makes CRISPR Cas systems convenient and efficient means for cutting various DNA sequences. The technique allows for simultaneous cutting of DNA at several regions using guide RNAs of different sequences. This approach is also used to simultaneously modify several genes in eukaryotic organisms.

By their nature, CRISPR-Cas systems are prokaryotic immune systems capable of highly specific introduction of breaks into a viral genetic material (Mojica F. J. M. et al. Intervening sequences of regularly spaced prokaryotic repeats derive from foreign genetic elements //Journal of molecular evolution. - 2005. - Vol. 60. - Issue 2. -pp. 174-182). The abbreviation CRISPR-Cas stands for "Clustered Regularly Interspaced Short Palindromic Repeats and CRISPR associated Genes" (Jansen R. et al. Identification of genes that are associated with DNA repeats in prokaryotes //Molecular microbiology. - 2002. - Vol. 43. - Issue 6. - pp. 1565-1575). All CRISPR-Cas systems consist of CRISPR cassettes and genes encoding various Cas proteins (Jansen R. et al., Molecular microbiology. - 2002. - Vol. 43. - Issue 6. - pp. 1565-1575). CRISPR cassettes consist of spacers, each having a unique nucleotide sequence, and repeated palindromic repeats (Jansen R. et al., Molecular microbiology. - 2002. - Vol. 43. - Issue 6. - pp. 1565-1575). The transcription of CRISPR cassettes followed by processing thereof results in the formation of guide crRNAs, which together with Cas proteins form an effector complex (Brouns S. J. J. et al. Small CRISPR RNAs guide antiviral defense in prokaryotes //Science. - 2008. - Vol. 321. - Issue 5891. - pp. 960-964). Due to the complementary pairing between the crRNA and a target DNA site, which is called the protospacer, Cas nuclease recognizes a DNA target and highly specifically introduces a break therein.

CRISPR-Cas systems with a single effector protein are grouped into six different types (types I-VI), depending on Cas proteins that are included in the systems. In 2013, it was proposed for the first time to use the Type II CRISPR-Cas9 system for editing the genomic DNA of human cells (Cong L, et al., Multiplex genome engineering using CRISPR/Cas systems. Science. 2013 Feb 15;339(6121 ):819-23). The type II CRISPR-Cas9 system is characterized in its simple composition and mechanism of activity, i.e. its functioning requires the formation of an effector complex consisting only of one Cas9 protein and two short RNAs as follows: crRNA and tracer RNA (tracrRNA). The tracer RNA complementarily pairs with a crRNA region, which originates from a CRISPR repeat, to form a secondary structure necessary for the binding of guide RNAs to the Cas effector. Determining the sequence of guide RNAs is an important step in the characterization of previously unstudied Cas orthologues. The Cas9 effector protein is an RNA-dependent DNA endonuclease with two nuclease domains (HNH and RuvC) that introduce breaks into the complementary strands of target DNA, thus producing a double-strand DNA break (Deltcheva E. et al. CRISPR RNA maturation by trans-encoded small RNA and host factor RNase III //Nature. - 2011. - Volume 471. - Issue 7340. - p. 602).

Thus far, several CRISPR-Cas nucleases are known that are capable of targeted and specific introduction of double-strand breaks into DNA. The CRISPR-Cas9 technology is one of the most modern and rapidly developing techniques for introducing breaks in DNA of various organisms, ranging from bacterial strains to human cells, also offering in vitro application (Song M. The CRISPR/Cas9 system: Their delivery, in vivo and ex vivo applications and clinical development by startups. Biotechnol Prog. 2017 Jul;33(4):1035-1045).

The effector ribonucleic complex consisting of Cas9 and a crRNA/tracrRNA duplex requires the presence of PAM (protospacer adjusted motif) on a DNA target for recognition and subsequent hydrolysis of DNA, in addition to crRNA spacer-protospacer complementarity. (Mojica F. J.M. et al. 2009). PAM is a strictly defined sequence of several nucleotides located in type II systems adjacent to or several nucleotides away from the 3' end of the protospacer on an off-target chain. In the absence of PAM, the hydrolysis of DNA bonds followed by the formation of a double-strand break does not take place. The need for the presence of a PAM sequence on a target increases recognition specificity but at the same time imposes restraints on the selection of target DNA regions for introducing a break. Thus, the presence of the desired PAM sequence flanking the DNA target from the 3'-end is a feature that limits the use of CRISPR-Cas systems at any DNA site.

Different CRISPR-Cas proteins use different, unique PAM sequences for the activity thereof. The use of CRISPR-Cas proteins with novel various PAM sequences is necessary to make it possible to modify any DNA region, both *in vitro* and in the genome of living organisms. Modification of eukaryotic genomes also requires the use of the small-sized nucleases to provide AAV-mediated delivery of CRISPR-Cas systems into cells.

Although a number of techniques for cutting DNA and modifying a genomic DNA sequence are known, there is still a need for novel effective means for modifying DNA in various organisms and at strictly specific sites of a DNA sequence.

### Summary of the invention

The object of the present invention is to provide novel means for modifying a genomic DNA sequence of unicellular or multicellular organisms using CRISPR-Cas9 systems. Currently existing systems are of limited use due to a specific PAM sequence that must be present at the 3' end of the DNA region to be modified. Search for novel Cas9 enzymes with other PAM sequences will expand the range of available means for the formation of a double-strand break at desired, strictly specific sites in DNA molecules of various organisms. To solve this problem, the authors characterized the previously predicted for *Pasteurella pneumotropica* (*P. pneumotropica*) the type II CRISPR nuclease PpCas9, which can be used to introduce directed modifications into the genome of both the above and other organisms. The present invention is characterized in that it has the following essential features: (a) short PAM sequence that is different from other known PAM sequences; (b) relatively small size of the characterized PpCas9 protein, which is 1055 amino acid residues (a.a.r.).

Said problem is solved by means of the use of a protein comprising the amino acid sequence of SEQ ID NO: 1 or comprising an amino acid sequence that is at least 95% identical to the amino acid sequence of SEQ ID NO: 1 and differs from SEQ ID NO: 1 only in non-conserved amino acid residues, to form, in DNA molecule, a double-strand break located immediately before the nucleotide sequence 5'-NNNN(A/G)TT-3' in said DNA molecule. In some embodiments of the invention, this use is characterized in that the double-strand break in the DNA molecule is formed at a temperature of 35 °C to 45 °C. In some embodiments of the invention, this use is characterized in that the double-strand break is formed in the genomic DNA of a mammalian cell. In some embodiments of the invention, this use is characterized in that the formation of the double-strand break in the DNA molecule results in the modification of the genomic DNA of said mammalian cell.

Said problem is further solved by providing a method for modifying a genomic DNA sequence in a cell of a unicellular or multicellular organism, comprising the introduction, into said cell of the organism, of an effective amount of: a) a protein comprising the amino acid sequence of SEQ ID NO: 1, or a nucleic acid encoding the protein comprising the amino acid sequence of SEQ ID NO: 1, and b) a guide RNA comprising a sequence that forms a duplex with the nucleotide sequence of an organism's genomic DNA region, which is directly adjacent to the nucleotide sequence 5'-NNNN(A/G)TT-3' and interacts with said protein following the formation of the duplex, or a DNA sequence encoding said guide RNA; wherein the interaction of said protein with the guide RNA and the nucleotide sequence 5'-NNNN(A/G)TT-3' results in the formation of a double-strand break in the genomic DNA sequence immediately adjacent to the sequence 5'-NNNN(A/G)TT-3'.

In some embodiments of the invention, the method is characterized in that it further comprises the introduction of an exogenous DNA sequence simultaneously with the guide RNA. In some embodiments of the invention, the method is characterized in that said cell is a mammalian cell.

A mixture of crRNA and tracer RNA (tracrRNA), which can form a complex with a target DNA region and PpCas9 protein, may be used as a guide RNA. In preferred embodiments of the invention, a hybrid RNA constructed based on crRNA and tracer RNA may be used as a guide RNA. Methods for constructing a hybrid guide RNA are known to those skilled (Hsu PD, et al., DNA targeting specificity of RNA-guided Cas9 nucleases. Nat Biotechnol. 2013 Sep;31(9):827-32). One of the approaches for constructing a hybrid RNA has been disclosed in the Examples below.

The invention may be used both for in vitro cutting target DNA, and for modifying the genome of some living organism. The genomic DNA may be modified in a direct fashion, i.e. by cutting the genomic DNA at a corresponding site, as well as by inserting an exogenous DNA sequence via homologous repair.

Any region of double-strand or single-strand DNA from the genome of an organism other than that used for administration (or a composition of such regions among themselves and with other DNA fragments) may be used as an exogenous DNA sequence, wherein said region (or composition of regions) is intended to be integrated into the place of a double-strand break in target DNA, induced by PpCas9 nuclease. In some embodiments of the invention, a region of double-strand DNA from the genomic DNA of an organism used for the introduction of PpCas9 protein, further modified by mutations (substitution of nucleotides), as well as by insertions or deletions of one or more nucleotides, may be used as an exogenous DNA sequence.

The technical result of the present invention is to increase the versatility of the available CRISPR-Cas9 systems to enable the use of Cas9 nuclease for cutting genomic or plasmid DNA in a larger number of specific sites and specific conditions. The novel nuclease may be used in the cells of bacteria, mammals or other organisms.

### Brief description of drawings

Fig. 1. Scheme of the locus of the CRISPR PpCas9 system. DR (direct repeat) is a regularly repeated region that is part of a CRISPR cassette.
Fig. 2. In vitro PAM screening. Scheme of the experiment.
Fig. 3. PpCas9 nuclease cutting of 7N library fragments at different reaction temperatures.
Fig. 4. (A) Analysis of the results of in vitro screening of PpCas9 nuclease using the calculation of the proportion change logarithm for each specific nucleotide at each PAM (FC) position. (B) PAM Logo of PpCas9 nuclease. The occurence of Adenine, Cytosine, Thymine, and Guanine is indicated for each position. The height of the letters corresponds to the occurrence of nucleotide at a given position of PAM sequence.
Fig. 5. Verification of the effect of single-nucleotide substitutions at PAM position 1 on the efficiency of cutting the DNA target by PpCas9 nuclease.
Fig. 6. Verification of the significance of nucleotide positions in the PpCas9 PAM sequence.
Fig. 7. Verification of the effect of A to G substitution at PAM position 5 on the efficiency of cutting of the DNA target by PpCas9 nuclease.
Fig. 8. Verification of the effect of single-nucleotide substitutions at PAM position 7 on the efficiency of cutting the DNA target by PpCas9 nuclease.
Fig. 9. Cutting of various DNA sites using the PpCas9 protein. Lanes 1 and 2 are positive controls.
Fig. 10. Verification of recognition of the PAM sequence CAGCATT by PpCas9 nuclease. Lanes 1 and 2 are positive controls.
Fig. 11. Diagram of the DNA cutting tool PpCas9.
Fig. 12. Experiment on cutting of a DNA target. Hybrid guide RNAs of different lengths were used.
Fig. 13. Alignment of amino acid sequences of PpCas9 and Cas9 proteins from *Staphylococcus aureus* using the NCBI BLASTp software (default parameters).
Fig. 14. Modification of the genomic DNA of human cells using PpCas9. (A) is the scheme of experiment to determine the efficiency of modifying the genomic DNA of human cells using a plasmid bearing PpCas9. (B) is the results of the analysis of insertions and deletions of nucleotides into the sequence of target sites of the genomic DNA of human cells (top - reaction products with T7 endonuclease I were applied onto agarose gel electrophoresis, bottom - examples of insertions and deletions formed by PpCas9 in the EMX1 gene which were determined by high throughput sequencing)

### Detailed disclosure of the invention

As used in the description of the present invention, the terms "includes" and "including" shall be interpreted to mean "includes, among other things". Said terms are not intended to be interpreted as "consists only of". Unless defined separately, the technical and scientific terms in this application have typical meanings generally accepted in the scientific and technical literature.

As used herein, the term "percent homology of two sequences" is equivalent to the term "percent identity of two sequences". Sequence identity is determined based on a reference sequence. Algorithms for sequence analysis are known in the art, such as BLAST described in Altschul et al., J. Mol. Biol., 215, pp. 403-10 (1990). For the purposes of the present invention, to determine the level of identity and similarity between nucleotide sequences and amino acid sequences, the comparison of nucleotide and amino acid sequences may be used, which is performed by the BLAST software package provided by the National Center for Biotechnology Information (http://www.ncbi.nlm.nih.gov/blast) using gapped alignment with standard parameters. Percent identity of two sequences is determined by the number of positions of identical amino acids in these two sequences, taking into account the number of gaps and the length of each gap to be entered for optimal comparison of the two sequences by alignment Percent identity is equal to the number of identical amino acids at given positions taking account of sequence alignment divided by the total number of positions and multiplied by 100.

The term "specifically hybridizes" refers to the association between two single-strand nucleic acid molecules or sufficiently complementary sequences, which permits such hybridization under predetermined conditions typically used in the art.

The phrase "a double-strand break located **immediately** before the nucleotide PAM sequence" means that a double-strand break in a target DNA sequence will be made at a distance of 0 to 25 nucleotides before the nucleotide PAM sequence.

An exogenous DNA sequence introduced simultaneously with a guide RNA is intended to refer to a DNA sequence prepared specifically for the specific modification of a double-strand target DNA at the site of break determined by the specificity of the guide RNA. Such a modification may be, for example, an insertion or deletion of certain nucleotides at the site of a break in target DNA. The exogenous DNA may be either a DNA region from a different organism or a DNA region from the same organism as that of target DNA.

A protein comprising a specific amino acid sequence is intended to refer to a protein having an amino acid sequence composed of said amino acid sequence and possibly other sequences linked by peptide bonds to said amino acid sequence. An example of other sequences may be a nuclear localization signal (NLS), or other sequences that provide increased functionality for said amino acid sequence.

An exogenous DNA sequence introduced simultaneously with a guide RNA is intended to refer to a DNA sequence prepared specifically for the specific modification of a double-strand target DNA at the site of break determined by the specificity of the guide RNA. Such a modification may be, for example, an insertion or deletion of certain nucleotides at the site of a break in target DNA. The exogenous DNA may be either a DNA region from a different organism or a DNA region from the same organism as that of target DNA.

An effective amount of protein and RNA introduced into a cell is intended to refer to such an amount of protein and RNA that, when introduced into said cell, will be able to form a functional complex, i.e. a complex that will specifically bind to target DNA and produce therein a double-strand break at the site determined by the guide RNA and PAM sequence on DNA. The efficiency of this process may be assessed by analyzing target DNA isolated from said cell using conventional techniques known to those skilled.

A protein and RNA may be delivered to a cell by various techniques. For example, a protein may be delivered as a DNA plasmid that encodes a gene of this protein, as an mRNA for translation of this protein in cell cytoplasm, or as a ribonucleoprotein complex that includes this protein and a guide RNA. The delivery may be performed by various techniques known to those skilled.

The nucleic acid encoding system's components may be introduced into a cell directly or indirectly as follows: by way of transfection or transformation of cells by methods known to those skilled, by way of the use of a recombinant virus, by way of manipulations on the cell, such as DNA microinjection, etc.

A ribonucleic complex consisting of a nuclease and guide RNAs and exogenous DNA (if necessary) may be delivered by way of transfecting the complexes into a cell or by way of mechanically introducing the complex into a cell, for example, by way of microinjection.

A nucleic acid molecule encoding the protein to be introduced into a cell may be integrated into the chromosome or may be an extrachromosomally replicating DNA. In some embodiments, to ensure effective expression of the protein gene with DNA introduced into a cell, it is necessary to modify the sequence of said DNA in accordance with the cell type in order to optimize the codons for expression, which is due to unequal frequencies of occurrence of synonymous codons in the coding regions of the genome of various organisms. Codon optimization is necessary to increase expression in animal, plant, fungal, or microorganism cells.

For a protein that has a sequence that is at least 95% identical to the amino acid sequence of SEQ ID NO: 1 to function in a eukaryotic cell, it is necessary for this protein to end up in the nucleus of this cell. Therefore, in some embodiments of the invention, a protein having a sequence that is at least 95% identical to the amino acid sequence of SEQ ID NO: 1 and which is further modified at one or both ends by the addition of one or more nuclear localization signals is used to form double-strand breaks in target DNA. For example, a nuclear localization signal from the SV40 virus may be used. To provide efficient delivery to the nucleus, the nuclear localization signal may be separated from the main protein sequence by a spacer sequence, for example, described in Shen B, et al. "Generation of gene-modified mice via Cas9/RNA-mediated gene targeting", Cell Res. 2013 May;23(5):720-3. Further, in other embodiments, a different nuclear localization signal or an alternative method for delivering said protein into the cell nucleus may be used.

The present invention encompasses the use of a protein from the *P. pneumotropica* organism, which is homologous to the previously characterized Cas9 proteins, to introduce double-strand breaks into DNA molecules at strictly specified positions. The use of CRISPR nucleases to introduce targeted modifications to the genome has a number of advantages. First, the specificity of the system's activity is determined by a crRNA sequence, which allows for the use of one type of nuclease for all target loci. Secondly, the technique enables the delivery of several guide RNAs complementary to different gene targets into a cell at once, thereby making it possible to simultaneously modify several genes at once.

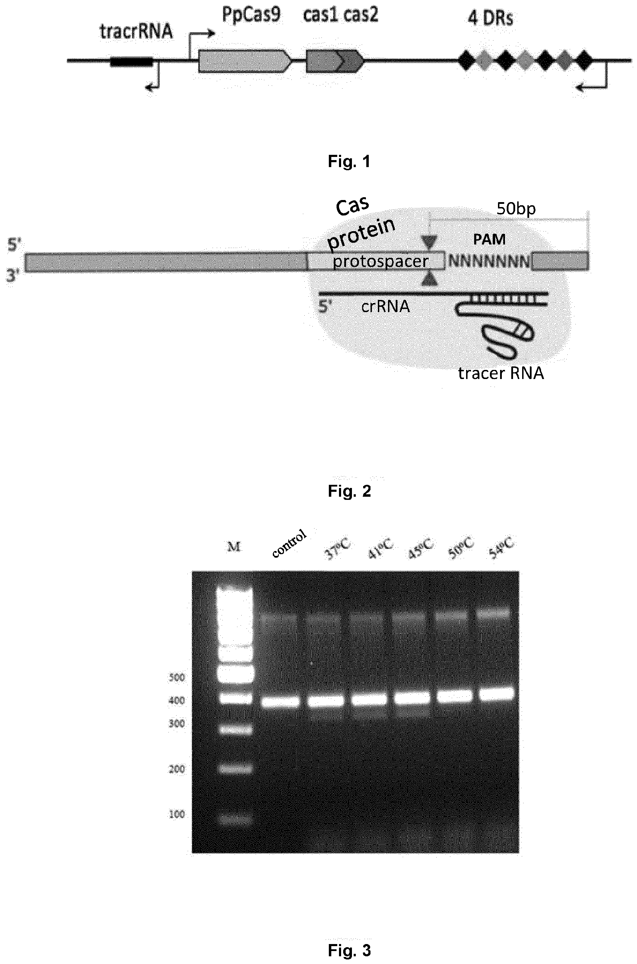

PpCas9 is a Cas nuclease found in *Pasteurella pneumotropica ATCC 35149*, a rodent pathogen that lives in the lungs of the animals. The *Pasteurella pneumotropica (P. pneumotropica)* CRISPR Cas9 system (hereinafter referred to as CRISPR PpCas9) belongs to type II-C CRISPR Cas systems and consists of a CRISPR cassette carrying four direct repeats (DR) with the sequence 5'ATTATAGCACTGCGAAATGAAAAAGGGAGCTACAAC3', interspaced by the sequences of unique spacers. None of the spacers of the system coincides in sequence with the currently known bacteriophages or plasmids, which fact makes it impossible to determine the PpCas9 PAM of interest by bioinformatic analysis. To the CRISPR cassette there are adjacent the gene for the effector Cas9 protein PpCas9, as well as the genes for the Cas1 and Cas2 proteins involved in adaptation and integration of new spacers. Nearby the Cas genes, a sequence was found partially complementary to direct repeats and folding into a characteristic secondary structure, which is contemplated to be the tracer RNA (tracrRNA) (Fig. 1)

Knowledge of the characteristic architecture of the RNA-Cas protein complex of type II-C systems made it possible to predict the direction of transcription of the CRISPR cassette: pre-crRNA is transcribed in the opposite direction to the Cas genes (Fig. 1)

Thus, the analysis of the sequence of the PpCas9 locus made it possible to predict the sequences of tracer and guide RNAs (Table 1).

**Table 1. Sequences of guide RNAs of the CRISPR PpCas9 system, which were determined by bioinformatics methods. Bold indicates the sequence of direct repeat, DR.**

| Name | Sequence |
|---|---|
| PpCas9 trRNA | 5'GCGAAATGAAAAACGUUGUUACAAUAAGAGAUGAAUUUCUCGCAAAG CTCUGCCUCUUGAAAUUUCGGUUUCAAGAGGCAUCUUUUU-3' |
| PpCas9 crRNA | 5'-xxxxxxxxxxxxxxxxxxxx**GUUGUAGCUCCCUUUUUCAUUUCGC-**3' |

To verify the activity of PpCas9 nuclease and determine the PpCas9 PAM of interest, we conducted experiments on recreating the DNA cutting reaction *in vitro.* To determine the PAM sequence of the PpCas9 protein, *in vitro* cutting of double-strand PAM libraries was employed. To this end, it was necessary to obtain all the components of the PpCas9 effector complex as follows: guide RNAs and a nuclease in a recombinant form. Determination of the guide RNA sequence made it possible to synthesize crRNA and tracrRNA molecules *in vitro.* The synthesis was carried out using the NEB HiScribe T7 RNA synthesis kit. The double-strand DNA libraries were 374 base pair (bp) fragments comprising a protospacer sequence flanked by randomized seven nucleotides (5'-NNNNNNN-3') from the 3' end: 5'-cccggggtaccacggagagatggtggaaatcatctttctcgtgggcatccttgatggccacctcgtcggaagtgcccacgaggatgacagcaatgc caatgctgggggggctcttctgagaacgagctctgctgcctgacacggccaggacggccaacaccaaccagaacttgggagaacagcactccgc tctgggcttcatcttcaactcgtcgactccctgcaaacacaaagaaagagcatgttaaaataggatctacatcacgtaacctgtcttagaagaggctag atactgcaattcaaggaccttatctcctttcattgagcac**NNNNNNN**aactccatcta ccagcctactctcttatctctggtatt -3'

To cut this target, guide RNAs of the following sequence were used:
tracrRNA: 
5'GCGAAATGAAAAACGUUGUUACAAUAAGAGAUGAAUUUCUCGCAAAGCTCUGCCUCU
UGAAAUUUCGGUUUCAAGAGGCAUCUUUUU
and crRNA: 5' **uaucuccuuucauugagcac**GUUGUAGCUCCCUUUUUCAUUUCGC.

Bold indicates the crRNA sequence that is complementary to the protospacer (target DNA sequence).

To produce a recombinant PpCas9 protein, the gene thereof was cloned into the plasmid pET21a. DNA synthesized by Integrated DNA Technologies (IDT) was used as the DNA encoding the gene. The sequence was codon-optimized to exclude rare codons found in the *P. pneumotropica* genome. *E. coli* Rosetta cells were transformed with the resulting plasmid pET21a-6xHis-PpCas9.

500 µl of overnight culture was diluted in 500 ml of LB medium, and the cells were grown at 37 °C until an optical density of 0.6 Ru was obtained. The synthesis of the target protein was induced by adding IPTG to a concentration of 1 mM, the cells were then incubated at 20 °C for 6 hours. Then, the cells were centrifuged at 5,000 g for 30 minutes, the resulting cellular precipitates were frozen at -20 °C.

The precipitates were thawed on ice for 30 minutes, resuspended in 15 ml of lysis buffer (Tris-HCI 50 mM pH 8, 500 mM NaCl, β-mercaptoethanol 1 mM, imidazole 10 mM) supplemented with 15 mg of lysozyme and re-incubated on ice for 30 minutes. The cells were then disrupted by sonication for 30 minutes and centrifuged for 40 minutes at 16,000 g. The resulting supernatant was passed through a 0.2 µm filter and applied onto a HisTrap HP 1 mL column (GE Healthcare) at 1 ml/min.

Chromatography was performed using the AKTA FPLC chromatograph (GE Healthcare) at 1 ml/min. The column with the applied protein was washed with 20 ml of lysis buffer supplemented with 30 mM imidazole, after which the protein was washed off with lysis buffer supplemented with 300 mM imidazole.

Then, the protein fraction obtained in the course of affinity chromatography was passed through a Superdex 200 10/300 GL gel filtration column (24 ml) equilibrated with the following buffer: Tris-HCI 50 mM pH 8, 500 mM NaCl, 1 mM DTT. Using an Amicon concentrator (with a 30 kDa filter), fractions corresponding to the monomeric form of the PpCas9 protein were concentrated to 3 mg/ml, after which the purified protein was stored at -80 °C in a buffer containing 10% glycerol.

The *in vitro* reaction of cutting the linear PAM libraries was carried out in a volume of 20 µl under the following conditions. The reaction mixture consisted of: 1× CutSmart buffer (NEB), 5 mM DTT, 100 nM PAM library, 2 µM trRNA/crRNA, 400 nM PpCas9 protein. As a control, samples containing no RNA were prepared in a similar way. The samples were incubated at different temperatures and analyzed by gel electrophoresis in 2% agarose gel. In the case of correct recognition and specific cutting of DNA by PpCas9 protein, two DNA fragments of about 326 and 48 base pairs should be generated (see Fig. 2).

The experiment results showed that PpCas9 has nuclease activity and cuts a portion of the PAM library fragments. The temperature gradient (Fig. 3) showed that the protein is active in the temperature range of 35-45 °C. The study then used a temperature of 42 °C as a working temperature.

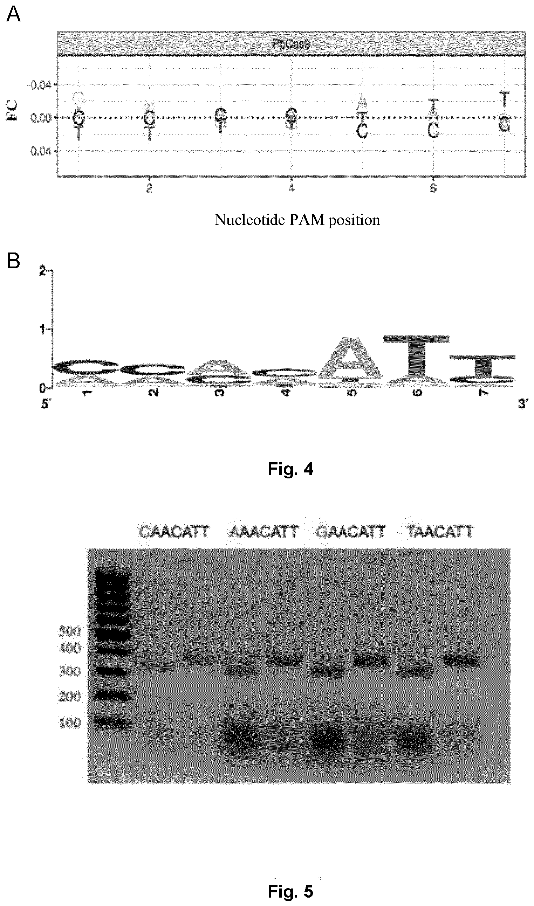

The library cutting reaction was repeated under the selected conditions. The reaction products were applied onto 1.5% agarose gel and subjected to electrophoresis. Uncut DNA fragments with a length of 374 bp were extracted from the gel and prepared for high-throughput sequencing using the NEB NextUltra II kit. The samples were sequenced on the lllumina platform, and then the analysis of the sequences was carried out using bioformatical methods: we determined the difference in occurrence of nucleotides at individual positions of PAM (NNNNNNN) as compared to the control sample using the approach described in (Maxwell CS, et al., A detailed cell-free transcription-translation-based assay to decipher CRISPR protospacer-adjacent motifs. Methods. 2018 Jul 1; 143:48-57). Furthermore, PAM logo was built to analyze the results (Fig. 4).

Both approaches to data analysis (Fig. 4) indicate the significance of PAM positions 5, 6 and 7. Thus, *in vitro* analysis allowed to establish the putative PAM sequence for PpCas9 as follows: NNNNATT. However, this sequence is only putative in view of inaccurate results obtained by screening approaches to determine PAM.

In this regard, the significance of individual PAM sequence positions was verified for more precise determination of the sequence. To this end, we performed in vitro reactions of cutting of DNA fragments containing a DNA target 5'-atctcctttcattgagcac-3' flanked by PAM sequence CAAC**ATT** (or derivatives thereof): 5'-cccggggtaccacggagagatggtggaaatcatctttctcgtgggcatccttgatggccacctcgtcggaagtgcccacgaggatgacagcaatgc caatgctgggggggctcttctgagaacgagctctgctgcctgacacggccaggacggccaacaccaaccagaacttgggagaacagcactccgc tctgggcttcatcttcaactcgtcgactccctgcaaacacaaagaaagagcatgttaaaataggatctacatcacgtaacctgtcttagaagaggctag atactgcaattcaaggaccttatctcctttcattgagcacCAAC**ATT**aactccatcta ccagcctactctcttatctctggtatt- 3'

All DNA cutting reactions were performed under the following conditions:
1×CutSmart buffer
400 nM PpCas9
20 nM DNA
2 µM crRNA
2 µM tracrRNA
Incubation time - 30 minutes, reaction temperature - 42 °C.

The substitution of PAM position 1 with all four possible nucleotide variants did not affect the efficiency of protein activity (Fig. 5).

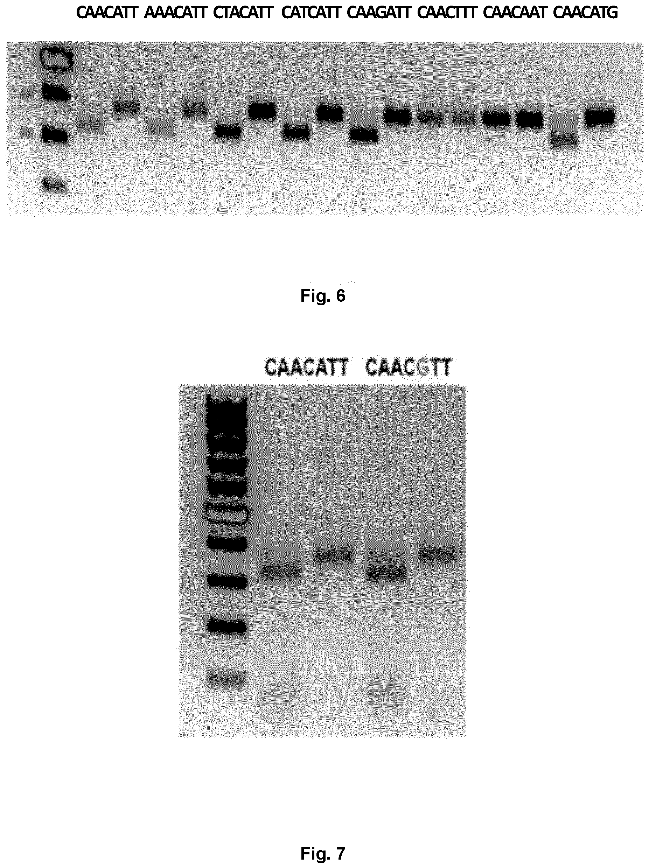

The predicted significance of positions 5 and 6 was confirmed experimentally by single nucleotide substitutions (purine with pyrimidine and vice versa) in each of the PAM positions. When the substitutions took place at positions 5 and 6, the protein practically stopped its activity. When the substitution took place at position 7, the efficiency of PpCas9 activity decreased twice, which fact reflects the reduced requirements for the nucleotide at this position (Fig. 6). Thus, according to the results of in vitro PAM screening of PpCas9 nuclease, the most probable nucleotides at PAM position 5 are adenine or guanine, which fact was confirmed experimentally (Fig. 7). A to G substitution did not reduce the efficiency of cutting of the fragment.

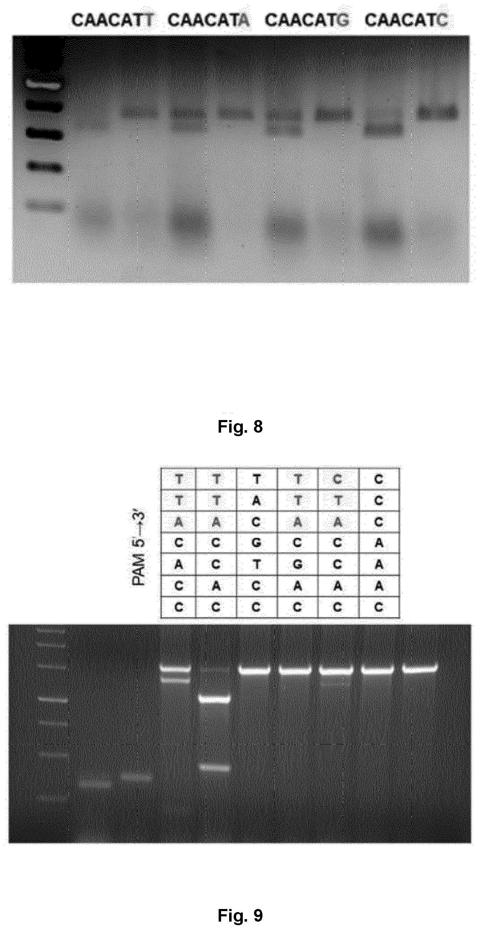

According to the results of in vitro screening, fragments with "T" or with "S" at position 7 should be recognized more efficiently. Additional experiments were conducted to definitively verify the significance of nucleotides at this position. The results of *in vitro* tests showed that substitution of the nucleotide "T" at position 7 with A or G reduced the cutting efficiency by 40-50% (Fig. 8). Thus, PAM position 7 is less conserved as compared to positions 5 and 6: purines at position 7 reduce recognition efficiency but do not prevent PpCas9 protein to introduce double-strand breaks into DNA.

The results of the study were as follows: PAM recognized by PpCas9 nuclease corresponds to the following formula 5'- NNNN(A/G)TT-3'. Position 7 is less conserved.

The following exemplary embodiments of the method are given for the purpose of disclosing the characteristics of the present invention and should not be construed as limiting in any way the scope of the invention.

### Example 1.Testing the activity ofPpCas9 protein in the cutting of various DNA targets.

In order to check the ability ofPpCas9 to recognize various DNA sequences flanked by the sequence 5'-NNNN(A/G)TT-3' , experiments were conducted on *in vitro* cutting of DNA targets from a human grin2b gene sequence (see Table 2).

**Table 2. DNA targets from the human GRIN2B gene.**

| sequence | PAM | | | | | | |
|---|---|---|---|---|---|---|---|
| TATCTCCTTTCATTGAGCAC | C | A | A | A | C | C | **C** |
| CAGCTGAAGTAATGTTAGAG | C | C | A | C | A | **T** | T |
| AATAAGAAAAACATTATTAT | C | A | C | C | A | T | T |
| GGGGCTATAAGTACACAAGC | C | C | T | G | C | A | T |
| CGTTCTCAGAAGAGCCCCCC | C | A | G | C | A | T | T |
| CCCACGAGAAAGATGATTTC | C | A | C | C | A | T | **C** |

A PCR fragment of the grin2b gene carrying recognition sites (Table 2) presumably recognizable by PpCas9 in accordance with PAM consensus sequence 5'-NNNN(A/G)TT-3' was used as a target in the cutting reaction. CrRNAs directing PpCas9 to these sites were synthesized to recognize these sequences.

The cutting reactions were performed under conditions selected for PpCas9; the result is shown in Fig. 9. Fig. 9 shows that the PpCas9 enzyme successfully cut three of the four targets with suitable PAM

The target on lane 6 had PAM sequence CAGCATT, which, according to the predictions based on the results of depletion analysis, should be efficiently recognized by the protein. However, the recognition of this fragment did not take place in this experiment.

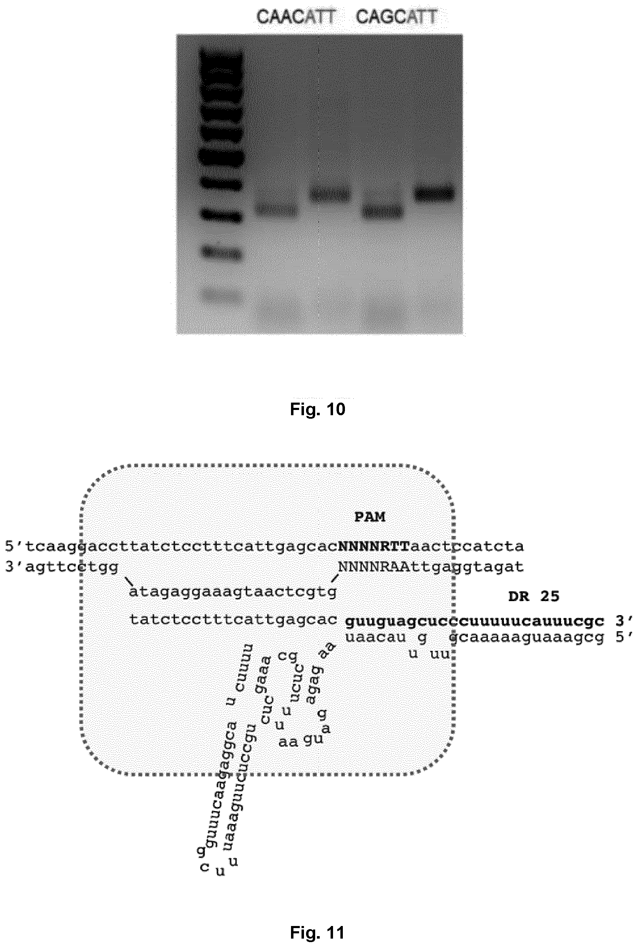

Therefore, the PAM CAGCATT was additionally verified on another protospacer target restricted to the same PAM (Fig. 10). In this case, the PAM was effectively recognized, which resulted in the cutting of DNA. Thus, the protein has some further preferences for the DNA target sequence. The preferences are possibly related to the secondary structure of DNA.

Thus, the studies showed the presence of nuclease activity in PpCas9, and also allowed to determine its PAM sequence and to verify the sequences of guide RNAs.

The PpCas9 ribonucleoprotein complex specifically introduces breaks in targets restricted to the PAM 5'-NNNN(A/G)TT -3' from the 5' end of the protospacer. The scheme of the PpCas9/RNA complex is shown in Fig. 11.

### Example 2. Use of hybrid guide RNA for cutting a DNA target.

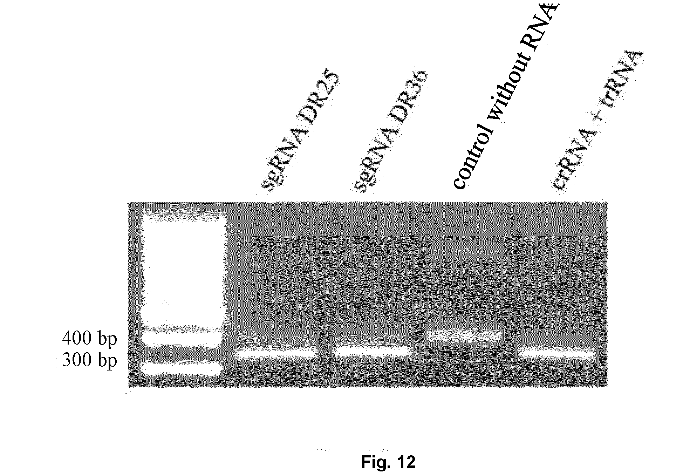

sgRNA is a form of guide RNAs, which is fused tracrRNA (tracer RNA) and crRNA. To select the optimal sgRNA, we constructed three variants of this sequence, which differed in the length of the tracrRNA-crRNA duplex. RNAs was synthesized in vitro and experiments involving them were conducted on cutting the DNA target (Fig. 12).
The following RNA sequences were used as hybrid RNAs:
1 - sgRNA1 25DR: 
UAUCUCCUUUCAUUGAGCACGUUGUAGCUCCCUUUUUCAUUUCGCGAAAGCGAAAUG
AAAAACGUUGUUACAAUAAGAGAUGAAUUUCUCGCAAAGCTCTGCCUCUUGAAAUUUC
GGUUUCAAGAGGCAUCUUUUU
2 - sgRNA2 36DR

UAUCUCCUUUCAUUGAGCACGUUGUAGCUCCCUUUUUCAUUUCGCAGUGCUAUAAU
GAAAAUUAUAGCACUGCGAAAUGAAAAACGUUGUUACAAUAAGAGAUGAAUUUCUCG
CAAAGCUCUGCCUCUUGAAAUUCGGUUUCAAGAGGCAUCUUUUU

Bold indicates a 20-nucleotide sequence that provides pairing with the DNA target (variable portion of sgRNA). Furthermore, the experiment used a control sample without RNA and a positive control, which is the cutting of the target using crRNA+trRNA.

A sequence containing the recognition site 5' tatctcctttcattgagcac 3' with the corresponding consensus sequence PAMCAACATT was used as a DNA target: 5'-cccggggtaccacggagagatggtggaaatcatctttctcgtgggcatccttgatggccacctcgtcggaagtgcccacgaggatgacagcaatgc caatgctgggggggctcttctgagaacgagctctgctgcctgacacggccaggacggccaacaccaaccagaacttgggagaacagcactccgc tctgggcttcatcttcaactcgtcgactccctgcaaacacaaagaaagagcatgttaaaataggatctacatcacgtaacctgtcttagaagaggctag atactgcaattcaaggacct**tatctcctttcattgagcac**CAACATTcaactccat ctaccagcctactctcttatctctggtatt - 3'

Bold indicates the recognition site, capital letters stand for PAM.

The reaction was performed under the following conditions: concentration of DNA sequence containing PAM (CAACATT) was 20 nM, protein concentration was 400 nM, RNA concentration was 2 µM; incubation time was 30 minutes, incubation temperature was 37 °C.

The selected sgRNA1 and sgRNA2 were found to be as efficient as the native tracrRNA and crRNA sequences: cutting took place in more than 80% of the DNA targets (Fig. 12).

These hybrid RNA variants may be used to cut any other target DNA after modifying the sequence that directly pairs with the DNA target.

### Example 3. Cas9 proteins from closely related organisms belonging to P. pneumotropica.

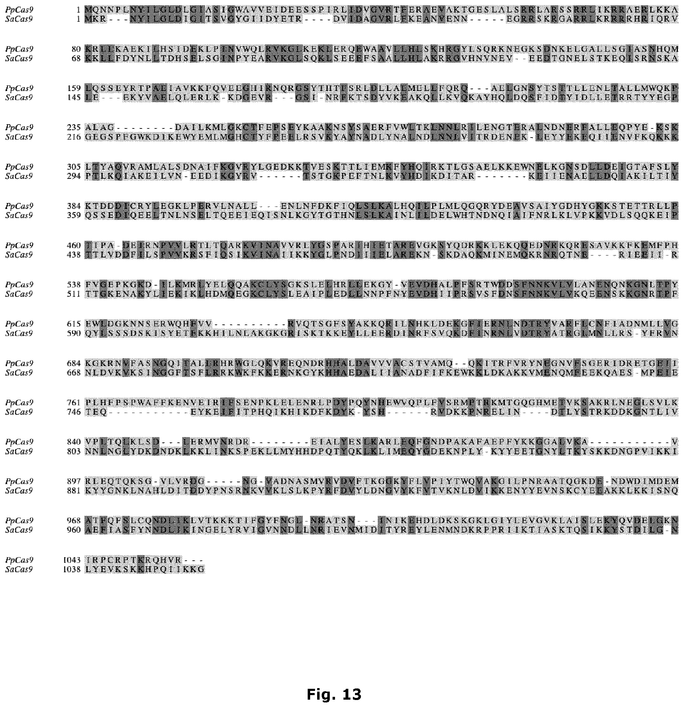

To date, no CRISPR-Cas9 enzymes have been characterized in *P. pneumotropica.* The Cas9 protein from *Staphylococcus aureus*, which is comparable in size, is identical to PpCas9 by 28% ((Fig. 13, the degree of identity was calculated by BLASTp software, default parameters). A similar degree of identity is present in other known Cas9 proteins (not shown).

Thus, PpCas9 protein differs significantly in its amino acid sequence from other Cas9 proteins studied to date.

Those skilled in the art of genetic engineering will appreciate that PpCas9 protein sequence variant obtained and characterized by the Applicant in the present description may be modified without changing the function of the protein itself (for example, by directed mutagenesis of amino acid residues that do not directly influence the functional activity (Sambrook et al., Molecular Cloning: A Laboratory Manual, (1989), CSH Press, pp. 15.3-15.108)). In particular, those skilled will recognize that non-conserved amino acid residues may be modified, without affecting the residues that are responsible for protein functionality (determining protein function or structure). Examples of such modifications include the substitutions of non-conserved amino acid residues with homologous ones. Some of the regions containing non-conserved amino acid residues are shown in Figure 12. In some embodiments of the invention, it is possible to use a protein comprising an amino acid sequence that is at least 95% identical to the amino acid sequence of SEQ ID NO: 1 and differs from SEQ ID NO: 1 only in non-conserved amino acid residues, to form, in DNA molecule, a double-strand break located immediately before the nucleotide sequence 5'-NNNN(A/G)TT-3' in said DNA molecule. Homologous proteins may be obtained by mutagenesis (for example, site-directed or PCR-mediated mutagenesis) of corresponding nucleic acid molecules, followed by testing the encoded modified Cas9 protein for the preservation of its functions in accordance with the functional analyses described herein.

### Example 4. Modification of the genomic DNA of human cells using PpCas9.

To modify the genomic DNA of human cells, the PpCas9 nuclease gene was cloned into a eukaryotic plasmid vector under the control of CMV promoter. Sequences encoding nuclear localization signals ensuring nuclease delivery to the cell nucleus were added to the 5' and 3' ends of the PpCas9 gene. The sgRNA sequence was cloned into the vector under the control of U6 promoter. To test the activity of the system, sgRNAs with a sequence complementary to target DNA of 20 and 24 nucleotide long were used. A similar plasmid bearing a SpCas9-based genomic DNA modification system known from the state of the art was used as a positive control. To assess the effectiveness of transfection, the plasmids further bore the GFP (green fluorescent protein) gene. The following regions of human genomic DNA were used as DNA targets (Table 3).

**Table 3. DNA targets of human EMX1 and GRIN2B genes.**

| **nuclease** | **Site name** | **Target sequence** | PAM |
|---|---|---|---|
| PpCas9 | EMX1.1 sg20 | GCCCTTCCTCCTCCAGCTTC | GTT |
| PpCas9 | EMX1.1 sg24 | TCAGGCCCTTCCTCCTCCAGCTTC | GTT |
| FpCas9 | EMX1.2 sg20 | GGAGGTGACATCGATGTCCT | ATT |
| FpCas9 | EMX1.2 sg24 | CATTGGAGGTGACATCGATGTCCT | ATT |
| PpCas9 | GRIN2B1.1 sg20 | CAGCTGAAGTAATGTTAGAG | ATT |
| PpCas9 | GRIN2B1.1 sg24 | TTAGCAGCTGAAGTAATGTTAGAG | ATT |
| PpCas9 | GRIN2B1.2 sg20 | AATAAGAAAAACATTATTAT | ATT |
| PpCas9 | GRIN2B1.2 sg24 | ATAAAATAAGAAAAACATTATTAT | ATT |
| SpCas9 | EMX1 sg20 | GAGTCCGAGCAGAAGAAGAA | GGG |
| SpCas9 | GRIN2B sg20 | ACCTTTTATTGCCTTGTTCA | AGG |

| | | | |
|---|---|---|---|
| EMX1.1 and EMX1.2 were two different modification sites in the EMX1 gene; similarly, GRIN2B1.1 and GRIN2B1.2 were two different modification sites in the GRIN2B gene. The DNA targets were flanked from the 3' end by the PAM sequences of PpCas9 5'-NNNNRTT -3' or SpCas9 5'- NGG -3'. | | | |

For the effective activity of PpCas9 nuclease in eukaryotic cells, it is necessary to import the protein into the nucleus of a eukaryotic cell. This may be done by way of using a nuclear localization signal from SV40 T-antigen (Lanford et al., Cell, 1986, 46: 575-582) linked to PpCas9 sequence via a spacer sequence described in Shen B, et al. "Generation of gene-modified mice via Cas9/RNA-mediated gene targeting", Cell Res. 2013 May;23(5):720-3 or without the spacer sequence.

In the given example, the complete amino acid sequence of the nuclease transported inside the nucleus of human cells was the following sequence:

MAPKKKRKVGIHGVPAAEQNNPLNYILGLDLGIASIGWAVVEIDEESSPIRLIDVGVRTFERAE
VAKTGESLALSRRLARSSRRLIKRRAERLKKAKRLLKAEKILHSIDEKLPINVWQLRVKGLKE
KLERQEWAAVLLHLSKHRGYLSQRKNEGKSDNKELGALLSGIASNHQMLQSSEYRTPAEIAV
KKFQVEEGHIRNQRGSYTHTFSRLDLLAEMELLFQRQAELGNSYTSTTLLENLTALLMWQKP
ALAGDAILKMLGKCTFEPSEYKAAKNSYSAERFVWLTKLNNLRILENGTERALNDNERFALL
EQPYEKSKLTYAQVRAMLALSDNAIFKGVRYLGEDKKTVESKTTLIEMKFYHQIRKTLGSAEL
KKEWNELKGNSDLLDEIGTAFSLYKTDDDICRYLEGKLPERVLNALLENLNFDKFIQLSLKAL
HQILPLMLQGQRYDEAVSAIYGDHYGKKSTETTRLLPTIPADEIRNPVVLRTLTQARKVINAV
VRLYGSPARIHIETAREVGKSYQDRKKLEKQQEDNRKQRESAVKKFKEMFPHFVGEPKGKDI
LKMRLYELQQAKCLYSGKSLELHRLLEKGYVEVDHALPFSRTWDDSFNNKVLVLANENQNK
GNLTPYEWLDGKNNSERWQHFVVRVQTSGFSYAKKQRILNHKLDEKGFIERNLNDTRYVAR
FLCNFIADNMLLVGKGKRNVFASNGQITALLRHRWGLQKVREQNDRHHALDAVVVACSTVA
MQQKITRFVRYNEGNVFSGERIDRETGEIIPLHFPSPWAFFKENVEIRIFSENPKLELENRLPDYP
QYNHEWVQPLFVSRMPTRKMTGQGHMETVKSAKRLNEGLSVLKVPLTQLKLSDLERMVNR
DREIALYESLKARLEQFGNDPAKAFAEPFYKKGGALVKAVRLEQTQKSGVLVRDGNGVADN
ASMVRVDVFTKGGKYFLVPIYTWQVAKGILPNRAATQGKDENDWDIMDEMATFQFSLCQND
LIKLVTKKKTIFGYFNGLNRATSNINIKEHDLDKSKGKLGIYLEVGVKLAISLEKYQVDELGKN
IRPCRPTKRQHVRFKRPAATKKAGQAKKKK

The plasmid used in this experiment had the following sequence:

gagggcctatttcccatgattccttcatatttgcatatacgatacaaggctgttagagagataattggaattaatttgactgtaaacacaaagat
attagtacaaaatacgtgacgtagaaagtaataatttcttgggtagtttgcagttttaaaattatgttttaaaatggactatcatatgcttaccgtaacttgaaa
gtatttcgatttcttggctttatatatcttgtggaaaggacgaaacaccgXXXXXXXXXXXXXXXXXXXXGTTGTAG
CTCCCTTTTTCATTTCGCGAAAGCGAAATGAAAAACGTTGTTACAATAAGAGATGAATTTC
TCGCAAAGCTCTGCCTCTTGAAATTTCGGTTTCAAGAGGCATCTTTTTtgctTCTCATGTCCA
ATATGACCGCCATGTTGACATTGATTATTGACTAGTTATTAATAGTAATCAATTACGGGGT
CATTAGTTCATAGCCCATATATGGAGTTCCGCGTTacataacttacggtaaatggcccgcctggctgaccgcccaa
cgacccccgcccattgacgtcaataatgacgtatgttcccatagtaacgccaatagggactttccattgacgtcaatgggtggagtatttacggtaaact
gcccacttggcagtacatcaagtgtatcatatgccaagtccgccccctattgacgtcaatgacggtaaatggcccgcctggcattatgcccagtacat
gaccttacgggactttcctacttggcagtacatctacgtattagtcatcgctattaccatggtgatgcggttttggcagtacaccaatgggcgtggatagc
ggtttgactcacggggatttccaagtctccaccccattgacgtcaatgggagtttgttttggcaccaaaatcaacgggactttccaaaatgtcgtaataa
ccccgccccgttgacgcaaatgggcggtaggcgtgtacggtgggaggtctatataagcAGAGCTCGTTTAGTGAACCGTCA
GAATTAATTCAGATCGATCTACCaccgccaccATGATGGCCCCAAAGAAGAAGCGGAAGGTCG
GTATCCACGGAGTCCCAGCAGCCGAACAGAATAATCCGCTTAACTACATTCTTGGGCT
GGATTTGGGAATTGCGAGTATAGGCTGGGCGGTGGTTGAGATCGATGAAGAGAGTA
GTCCGATACGCCTTATCGACGTTGGAGTTAGGACGTTCGAGAGGGCGGAGGTCGCC
AAGACCGGTGAGAGCTTGGCCCTCAGCCGGCGGCTCGCTCGATCTAGTCGCAGGCT
TATAAAGAGGAGGGCTGAGCGCCTTAAGAAAGCTAAGAGGCTCCTTAAGGCAGAAA
AAATTCTGCATAGTATCGACGAAAAGCTGCCGATAAATGTTTGGCAGCTCCGAGTAA
AAGGGCTGAAGGAAAAATTGGAAAGGCAGGAGTGGGCGGCGGTACTGCTTCATCTC
TCCAAGCACCGGGGCTATCTGTCTCAGCGAAAAACGAAGGTAAGTCAGACAACAA
GGAGCTGGGCGCACTTTTGTCCGGGATAGCGTCAAATCATCAGATGCTCCAATCAAG
TGAGTATCGGACCCCTGCGGAGATCGCCGTTAAAAAGTTTCAAGTTGAGGAGGGCC

ACATCAGAAATCAGAGGGGGTCTTACACCCATACGTTCTCTAGACTCGACCTCCTTG
CGGAAATGGAACTCCTGTTTCAGCGCCAGGCGGAGCTTGGTAACTCCTACACGTCCA
CTACCCTCCTGGAAAACCTGACAGCCCTGCTGATGTGGCAGAAGCCCGCTTTGGCG
GGGGATGCCATCCTGAAGATGCTGGGTAAATGCACCTTTGAGCCGTCAGAATATAAA
GCCGCCAAGAATAGTTACTCTGCGGAGCGATTTGTTTGGTTGACAAAGTTGAATAAC
CTGCGCATCCTGGAGAACGGTACCGAGCGCGCACTCAATGATAATGAGCGCTTCGC
CCTCCTGGAACAGCCCTACGAGAAGTCCAAGCTCACCTACGCCCAAGTCAGAGCCAT
GCTGGCTCTTAGTGACAACGCGATTTTTAAGGGCGTGCGATACTTGGGCGAGGATA
AGAAAACCGTAGAGTCAAAAACGACTCTGATCGAGATGAAATTCTATCACCAAATTA
GAAAGACCCTCGGTTCTGCCGAGCTGAAAAAGGAATGGAACGAACTTAAGGGTAAC
AGCGACCTGCTCGATGAAATCGGTACCGCATTTAGCCTTTATAAAACGGACGACGAC
ATCTGCCGATATTTGGAGGGGAAGCTCCCAGAGCGAGTATTGAATGCACTCCTTGAG
AACCTTAATTTTGACAAGTTCATTCAGCTGTCCCTCAAAGCACTGCATCAAATCCTCC
CACTTATGCTGCAAGGACAACGATACGACGAAGCCGTCAGCGCGATATATGGAGAT
CATTACGGAAAAAAGTCCACCGAGACCACACGACTGCTTCCTACGATCCCCGCCGAT
GAGATCAGAAATCCCGTAGTCCTTCGAACACTTACTCAGGCTAGGAAGGTGATTAAT
GCGGTAGTTAGGTTGTATGGATCTCCGGCACGGATACATATAGAAACAGCTCGCGA
AGTGGGTAAATCTTACCAAGACCGCAAGAAATTGGAGAAACAACAGGAGGATAACC
GAAAGCAACGAGAATCTGCCGTTAAAAAGTTTAAGGAAATGTTTCCTCACTTTGTAG
GAGAACCGAAGGGTAAAGATATCTTGAAAATGCGGTTGTACGAGTTGCAGCAAGCT
AAGTGTCTCTATAGCGGCAAGAGTTTGGAATTGCACCGCCTCCTGGAGAAAGGCTAC
GTGGAAGTAGACCATGCGCTCCCGTTTTCCCGAACCTGGGATGATTCTTTCAATAAC
AAAGTCCTTGTGCTGGCAAATGAGAACCAGAACAAAGGAAATCTGACTCCTTATGAG
TGGTTGGATGGCAAGAATAATTCTGAGCGGTGGCAACATTTCGTTGTCCGCGTCCAA
ACGTCAGGGTTCAGCTATGCTAAGAAACAAAGGATCCTCAATCACAAGCTCGACGAG
AAAGGATTCATAGAACGAAATTTGAATGACACTAGGTATGTGGCTCGATTTCTCTGC
AATTTTATTGCTGACAATATGCTCCTCGTTGGGAAGGGAAAGCGGAATGTTTTTGCA
TCAAATGGGCAGATAACGGCGCTCTTGAGACATAGATGGGGGCTGCAAAAGGTGAG
AGAGCAAAATGATAGACATCACGCCCTGGATGCCGTTGTAGTCGCCTGTTCAACGGT
TGCGATGCAGCAAAAGATCACTCGGTTCGTTAGGTATAACGAAGGAACGTTTTTAG
TGGAGAGCGCATAGATCGGGAAACAGGCGAAATCATCCCTTTGCATTTCCCAAGTCC
TTGGGCTTTTTCAAAGAGAATGTGGAAATAAGGATATTCAGTGAAAACCCTAAGTT
GGAGCTTGAGAATCGGTTGCCCGATTATCCCCAGTACAATCATGAGTGGGTTCAACC
GCTGTTCGTATCCCGCATGCCAACCCGAAAGATGACCGGGCAGGGTCACATGGAGA
CTGTGAAATCTGCAAAGAGACTTAATGAGGGCCTGTCAGTGTTGAAGGTGCCCTTGA
CTCAACTGAAATTGAGCGACCTCGAGCGCATGGTAAACCGCGATAGAGAAATCGCA
CTTTATGAGAGTCTGAAGGCGCGATTGGAACAATTCGGTAATGATCCGGCAAAGGCT
TTCGCTGAGCCATTCTACAAGAAGGGTGGAGCGCTGGTTAAGGCTGTCCGACTCGA
ACAGACACAAAAGTCAGGGGTCTTGGTCAGAGATGGTAACGGGGTTGCCGACAACG
CCTCCATGGTACGAGTAGATGTTTTCACGAAAGGAGGAAAATACTTTCTGGTACCTA
TCTATACCTGGCAAGTTGCCAAGGGAATACTCCCGAATAGGGCGGCGACCCAGGGA
AAGGATGAAAACGACTGGGATATAATGGATGAAATGGCTACGTTTCAGTTTAGCTTG
TGCCAGAATGACCTCATAAAACTGGTAACCAAAAAAAAGACTATATTCGGGTATTTC
AATGGCCTTAATCGGGCAACTTCCAATATCAACATCAAGGAACATGATCTGGATAAG
AGCAAGGGAAAGCTTGGTATCTATCTCGAAGTTGGAGTCAAGCTCGCTATTTCCCTC
GAGAAATATCAAGTAGATGAACTGGGAAAGAATATACGGCCATGCCGGCCCACAAA
AAGACAACACGTACGGTTCAAAAGGCCGGCGGCCACGAAAAAGGCCGGCCAGGCAAAA
AAGAAAAAGGGATCCTACCCATACGATGTTCCAGATTACGCTTATCCCTACGACGTGCCT
GATTATGCATACCCATATGATGTCCCCGACTATGCCGGCGCAACAAACTTCTCTCTGCTGA
AACAAGCCGGAGATGTCGAAGAGAATCCTGGACCGgtgagcaagggcgaggagctgttcaccggggtggtgcc
catcctggtcgagctggacggcgacgtaaacggccacaagttcagcgtgtccggcgagggcgagggcgatgccacctacggcaagctgaccct
gaagttcatctgcaccaccggcaagctgcccgtgccctggcccaccctcgtgaccaccctgacctacggcgtgcagtgcttcagccgctaccccga

ccacatgaagcagcacgacttcttcaagtccgccatgcccgaaggctacgtccaggagcgcaccatcttcttcaaggacgacggcaactacaagac
ccgcgccgaggtgaagttcgagggcgacaccctggtgaaccgcatcgagctgaagggcatcgacttcaaggaggacggcaacatcctggggca
caagctggagtacaactacaacagccacaacgtctatatcatggccgacaagcagaagaacggcatcaaggtgaacttcaagatccgccacaaca
tcgaggacggcagcgtgcagctcgccgaccactaccagcagaacacccccatcggcgacggccccgtgctgctgcccgacaaccactacctga
gcacccagtccgccctgagcaaagaccccaacgagaagcgcgatcacatggtcctgctggagttcgtgaccgccgccgggatcactctcggcat
ggacgagctgtacaagTAA

The following portions were distinguished in the plasmid sequence: the U6 promoter (the first region, capital letters), the sequence complementary to the protospacer ("XXX-XXX"), the conserved portion of sgRNA (the third region, capital letters), the PpCas9 gene (highlighted in bold), the GFP gene (the last region, capital letters).

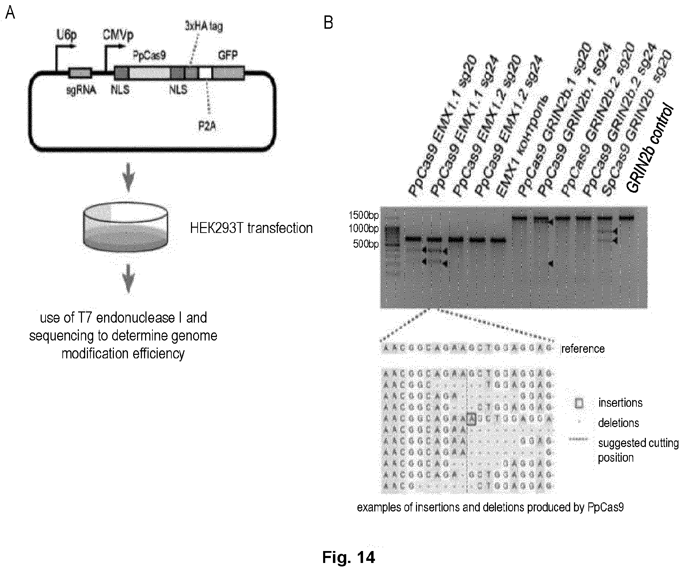

Plasmids with PpCas9 or SpCas9 were transfected into human HEK293T cell culture using the Lipofectamine 2000 reagent. 72 hours following transfection the cells were lysed, the resulting lysates were subjected to PCR to generate regions that include target modification sites of genomic DNA. The resulting PCR fragments were subjected to in vitro reaction with T7 endonuclease I to determine the frequency of insertions and deletions in the target sites of genomic DNA. The reaction products were applied onto agarose gel and subjected to electrophoresis. Fig. 14A shows that PpCas9 actively introduces modifications to the EMX1 and GRIN2b genes, with an efficiency similar to that of the SpCas9 nuclease described in the prior art.

This experiment showed that, in order to effectively modify genomic DNA, PpCas9 requires elongated sgRNAs as compared to those of SpCas9: in the given example, the efficiency of genetic modifications is greater when using sgRNA with a sequence complementary to DNA target having a length of 24 nucleotides (as compared to a length of 20 nucleotides).

High-throughput sequencing confirmed the introduced modifications in the target DNA sites. Fig. 14B shows examples of detectable modifications to the nucleotide sequence of the EMX1 gene.

Delivery in the form of a ribonucleic complex may also be employed to deliver NLS_PpCas9_NLS to human cells. It is carried out by incubating a recombinant form of PpCas9 NLS with guide RNAs in the CutSmart buffer (NEB). The recombinant protein is produced from bacterial producer cells by purifying the former by affinity chromatography (NiNTA, Qiagen) with size exclusion (Superdex 200).

The protein is mixed with RNAs in a ratio of 1:2 (PpCas9 NLS : sgRNA), the mixture is incubated for 10 minutes at room temperature, and then transfected into the cells.

Next, the DNA extracted therefrom is analyzed for inserts/deletions at the target DNA site (as described above).

The PpCas9 nuclease from the bacterium ***Pasteurella pneumotropica*** characterized in the present invention may be delivered, for modifying DNA, to cells of various origins using standard approaches and methods known to those skilled. PpCas9 has a number of advantages over previously characterized Cas9 proteins.

PpCas9 has a short, two-letter PAM, distinct from other known Cas nucleases, that is required for the system to function. The invention has shown that the presence of a short PAM (RTT) located 4 nucleotides away from the protospacer is sufficient for PpCas9 to successfully function in vivo.

The many small-sized Cas nucleases known thus far, which are capable of introducing double-strand breaks into DNA, have complex multi-letter PAM sequences, limiting the choice of sequences suitable for cutting. Among the Cas nucleases studied to date, which recognize short PAMs, only PpCas9 can recognize sequences flanked by the RTT motif.

The second advantage of PpCas9 is the small protein size (1055 aar). To date, it is the only small-sized protein studied that has a three-letter RTT PAM sequence.

PpCas9 is a novel, small-sized Cas nuclease with a short, easy-to-use PAM that differs from the currently known PAM sequences of other nucleases. The PpCas9 protein cuts various DNA targets with high efficiency, including genomic DNA in human cells at 37 °C, and may become the basis for a new genomic editing tool.

Although the invention has been described with reference to the disclosed embodiments, those skilled in the art will appreciate that the particular embodiments described in detail have been provided for the purpose of illustrating the present invention and are not be construed as in any way limiting the scope of the invention. It will be understood that various modifications may be made without departing from the spirit of the present invention.

## Claims

1. Use of a protein comprising the amino acid sequence of SEQ ID NO: 1 or comprising an amino acid sequence that is at least 95% identical to the amino acid sequence of SEQ ID NO: 1 and differs from SEQ ID NO: 1 only in non-conserved amino acid residues, to form, in DNA molecule, a double-strand break located immediately before the nucleotide sequence 5'-NNNN(A/G)TT-3' in said DNA molecule.

2. The use according to claim 1, **characterized in that** the double-strand break in the DNA molecule is formed at a temperature of 35 °C to 45 °C.

3. The use of the protein according to claim 1, wherein the protein comprises the amino acid sequence of SEQ ID NO: 1.

4. The use according to claim 1, **characterized in that** the double-strand break in the DNA molecule is formed in the genomic DNA of a mammalian cell.

5. The use according to claim 4, **characterized in that** the double-strand break in the DNA molecule leads to the modification of the genomic DNA of said mammalian cell.

6. A method for modifying a genomic DNA sequence in a cell of a unicellular or multicellular organism comprising the genomic DNA, said method including the introduction, into said cell of the organism, of an effective amount of: a) a protein comprising the amino acid sequence of SEQ ID NO: 1, or a nucleic acid encoding the protein comprising the amino acid sequence of SEQ ID NO: 1, and b) a guide RNA comprising a sequence that forms a duplex with the nucleotide sequence of an organism's genomic DNA region, which is directly adjacent to the nucleotide sequence 5'-NNNN(A/G)TT-3' and interacts with said protein following the formation of the duplex, or a DNA sequence encoding said guide RNA;
wherein the interaction of said protein with the guide RNA and the nucleotide sequence 5'-NNNN(A/G)TT-3' results in the formation of a double-strand break in the genomic DNA sequence immediately adjacent to the sequence 5'-NNNN(A/G)TT-3'.

7. The method according to claim 6, further comprising the introduction of an exogenous DNA sequence simultaneously with the guide RNA.

8. The method according to claim 6, **characterized in that** said cell is a mammalian cell.


Sequence listing

<110> JSC BIOCAD
<120> USE OF CAS9 PROTEIN FROM THE BACTERIUM PASTEURELLA PNEUMOTROPICA

<150> RU 2019136164
<151> 2019-11-11

<160> 2

<210> 1
<211> 1055
<212> PRT
<213> Pasteurella pneumotropica

<220>
<223> protein homologous to Cas9

<400> 1
Met Gln Asn Asn Pro Leu Asn Tyr Ile Leu Gly Leu Asp Leu Gly Ile
1               5                   10                  15
Ala Ser Ile Gly Trp Ala Val Val Glu Ile Asp Glu Glu Ser Ser Pro
            20                  25                  30
Ile Arg Leu Ile Asp Val Gly Val Arg Thr Phe Glu Arg Ala Glu Val
        35                  40                  45
Ala Lys Thr Gly Glu Ser Leu Ala Leu Ser Arg Arg Leu Ala Arg Ser
    50                  55                  60
Ser Arg Arg Leu Ile Lys Arg Arg Ala Glu Arg Leu Lys Lys Ala Lys
65                  70                  75                  80
Arg Leu Leu Lys Ala Glu Lys Ile Leu His Ser Ile Asp Glu Lys Leu
                85                  90                  95
Pro Ile Asn Val Trp Gln Leu Arg Val Lys Gly Leu Lys Glu Lys Leu
            100                 105                 110
Glu Arg Gln Glu Trp Ala Ala Val Leu Leu His Leu Ser Lys His Arg
        115                 120                 125
Gly Tyr Leu Ser Gln Arg Lys Asn Glu Gly Lys Ser Asp Asn Lys Glu
    130                 135                 140
Leu Gly Ala Leu Leu Ser Gly Ile Ala Ser Asn His Gln Met Leu Gln
145                 150                 155                 160
Ser Ser Glu Tyr Arg Thr Pro Ala Glu Ile Ala Val Lys Lys Phe Gln
                165                 170                 175
Val Glu Glu Gly His Ile Arg Asn Gln Arg Gly Ser Tyr Thr His Thr
            180                 185                 190
Phe Ser Arg Leu Asp Leu Leu Ala Glu Met Glu Leu Leu Phe Gln Arg
        195                 200                 205
Gln Ala Glu Leu Gly Asn Ser Tyr Thr Ser Thr Thr Leu Leu Glu Asn
    210                 215                 220
Leu Thr Ala Leu Leu Met Trp Gln Lys Pro Ala Leu Ala Gly Asp Ala
225                 230                 235                 240
Ile Leu Lys Met Leu Gly Lys Cys Thr Phe Glu Pro Ser Glu Tyr Lys
                245                 250                 255
Ala Ala Lys Asn Ser Tyr Ser Ala Glu Arg Phe Val Trp Leu Thr Lys
            260                 265                 270
Leu Asn Asn Leu Arg Ile Leu Glu Asn Gly Thr Glu Arg Ala Leu Asn
        275                 280                 285
Asp Asn Glu Arg Phe Ala Leu Leu Glu Gln Pro Tyr Glu Lys Ser Lys
    290                 295                 300
Leu Thr Tyr Ala Gln Val Arg Ala Met Leu Ala Leu Ser Asp Asn Ala
305                 310                 315                 320
Ile Phe Lys Gly Val Arg Tyr Leu Gly Glu Asp Lys Lys Thr Val Glu
                325                 330                 335
Ser Lys Thr Thr Leu Ile Glu Met Lys Phe Tyr His Gln Ile Arg Lys

            340                 345                 350
Thr Leu Gly Ser Ala Glu Leu Lys Lys Glu Trp Asn Glu Leu Lys Gly
        355                 360                 365
Asn Ser Asp Leu Leu Asp Glu Ile Gly Thr Ala Phe Ser Leu Tyr Lys
    370                 375                 380
Thr Asp Asp Asp Ile Cys Arg Tyr Leu Glu Gly Lys Leu Pro Glu Arg
385                 390                 395                 400
Val Leu Asn Ala Leu Leu Glu Asn Leu Asn Phe Asp Lys Phe Ile Gln
                405                 410                 415
Leu Ser Leu Lys Ala Leu His Gln Ile Leu Pro Leu Met Leu Gln Gly
            420                 425                 430
Gln Arg Tyr Asp Glu Ala Val Ser Ala Ile Tyr Gly Asp His Tyr Gly
        435                 440                 445
Lys Lys Ser Thr Glu Thr Thr Arg Leu Leu Pro Thr Ile Pro Ala Asp
    450                 455                 460
Glu Ile Arg Asn Pro Val Val Leu Arg Thr Leu Thr Gln Ala Arg Lys
465                 470                 475                 480
Val Ile Asn Ala Val Val Arg Leu Tyr Gly Ser Pro Ala Arg Ile His
                485                 490                 495
Ile Glu Thr Ala Arg Glu Val Gly Lys Ser Tyr Gln Asp Arg Lys Lys
            500                 505                 510
Leu Glu Lys Gln Gln Glu Asp Asn Arg Lys Gln Arg Glu Ser Ala Val
        515                 520                 525
Lys Lys Phe Lys Glu Met Phe Pro His Phe Val Gly Glu Pro Lys Gly
    530                 535                 540
Lys Asp Ile Leu Lys Met Arg Leu Tyr Glu Leu Gln Gln Ala Lys Cys
545                 550                 555                 560
Leu Tyr Ser Gly Lys Ser Leu Glu Leu His Arg Leu Leu Glu Lys Gly
                565                 570                 575
Tyr Val Glu Val Asp His Ala Leu Pro Phe Ser Arg Thr Trp Asp Asp
            580                 585                 590
Ser Phe Asn Asn Lys Val Leu Val Leu Ala Asn Glu Asn Gln Asn Lys
        595                 600                 605
Gly Asn Leu Thr Pro Tyr Glu Trp Leu Asp Gly Lys Asn Asn Ser Glu
    610                 615                 620
Arg Trp Gln His Phe Val Val Arg Val Gln Thr Ser Gly Phe Ser Tyr
625                 630                 635                 640
Ala Lys Lys Gln Arg Ile Leu Asn His Lys Leu Asp Glu Lys Gly Phe
                645                 650                 655
Ile Glu Arg Asn Leu Asn Asp Thr Arg Tyr Val Ala Arg Phe Leu Cys
            660                 665                 670
Asn Phe Ile Ala Asp Asn Met Leu Leu Val Gly Lys Gly Lys Arg Asn
        675                 680                 685
Val Phe Ala Ser Asn Gly Gln Ile Thr Ala Leu Leu Arg His Arg Trp
    690                 695                 700
Gly Leu Gln Lys Val Arg Glu Gln Asn Asp Arg His His Ala Leu Asp
705                 710                 715                 720
Ala Val Val Val Ala Cys Ser Thr Val Ala Met Gln Gln Lys Ile Thr
                725                 730                 735
Arg Phe Val Arg Tyr Asn Glu Gly Asn Val Phe Ser Gly Glu Arg Ile
            740                 745                 750
Asp Arg Glu Thr Gly Glu Ile Ile Pro Leu His Phe Pro Ser Pro Trp
        755                 760                 765
Ala Phe Phe Lys Glu Asn Val Glu Ile Arg Ile Phe Ser Glu Asn Pro
    770                 775                 780
Lys Leu Glu Leu Glu Asn Arg Leu Pro Asp Tyr Pro Gln Tyr Asn His
785                 790                 795                 800
Glu Trp Val Gln Pro Leu Phe Val Ser Arg Met Pro Thr Arg Lys Met
                805                 810                 815
Thr Gly Gln Gly His Met Glu Thr Val Lys Ser Ala Lys Arg Leu Asn
            820                 825                 830
Glu Gly Leu Ser Val Leu Lys Val Pro Leu Thr Gln Leu Lys Leu Ser
        835                 840                 845

Asp Leu Glu Arg Met Val Asn Arg Asp Arg Glu Ile Ala Leu Tyr Glu
    850                 855                 860
Ser Leu Lys Ala Arg Leu Glu Gln Phe Gly Asn Asp Pro Ala Lys Ala
865                 870                 875                 880
Phe Ala Glu Pro Phe Tyr Lys Lys Gly Gly Ala Leu Val Lys Ala Val
                885                 890                 895
Arg Leu Glu Gln Thr Gln Lys Ser Gly Val Leu Val Arg Asp Gly Asn
            900                 905                 910
Gly Val Ala Asp Asn Ala Ser Met Val Arg Val Asp Val Phe Thr Lys
        915                 920                 925
Gly Gly Lys Tyr Phe Leu Val Pro Ile Tyr Thr Trp Gln Val Ala Lys
    930                 935                 940
Gly Ile Leu Pro Asn Arg Ala Ala Thr Gln Gly Lys Asp Glu Asn Asp
945                 950                 955                 960
Trp Asp Ile Met Asp Glu Met Ala Thr Phe Gln Phe Ser Leu Cys Gln
                965                 970                 975
Asn Asp Leu Ile Lys Leu Val Thr Lys Lys Lys Thr Ile Phe Gly Tyr
            980                 985                 990
Phe Asn Gly Leu Asn Arg Ala Thr Ser Asn Ile Asn Ile Lys Glu His
        995                 1000                1005
Asp Leu Asp Lys Ser Lys Gly Lys Leu Gly Ile Tyr Leu Glu Val Gly
    1010                1015                1020
Val Lys Leu Ala Ile Ser Leu Glu Lys Tyr Gln Val Asp Glu Leu Gly
1025                1030                1035                1040
Lys Asn Ile Arg Pro Cys Arg Pro Thr Lys Arg Gln His Val Arg
                1045                1050                1055

<210> 2
<211> 3168
<212> DNA
<213> Pasteurella pneumotropica


<220>
<223> protein homologous to Cas9

<400> 2
atgcaaaata atccattaaa ttacatttta gggttagatt taggcattgc ttctattggt      60

tgggcggttg tggaaattga tgaggagagt tcacctattc gcttaattga tgtgggcgtc     120

cgtacatttg aacgggctga agtcgctaaa accggcgaaa gtttagcatt gtctcgtcgt     180

ttagctcgtt catcacggcg attaattaaa cgccgagcag agcgattaaa aaaagcaaaa     240

cgtttattaa aagcagaaaa gattttacat tctattgatg aaaaattacc cattaatgtt     300

tggcagcttc gagtaaaagg attgaaggaa aaactcgaac gtcaggagtg ggcagcggtt     360

ttattacatt tgtcaaagca tcgtggctat ttatcacaac gtaaaaatga gggtaaaagt     420

gataataaag agctgggggc attactttca ggtatcgcaa gtaaccacca aatgttgcaa     480

tcctccgaat atcgtacccc tgcagaaatt gcagtcaaaa aatttcaagt agaagaagga     540

catattcgta atcaacgtgg atcttatacc cataccttta gccgtttgga tttgttggca     600

gaaatggaat tattatttca acgccaagct gagttaggca attcttacac gtccaccaca     660

ttattagaaa atttgacggc gttactaatg tggcaaaagc cagctcttgc gggtgatgcg     720

attttaaaaa tgttgggcaa gtgtaccttc gaacccagcg aatataaagc cgcaaaaaat     780

agttattctg ctgaacgttt tgtgtggtta accaagctga ataatttacg cattttagaa 840

aatggcacgg aaagagcttt aaatgacaat gaacgttttg ctttgcttga gcaaccgtat 900

gagaaatcaa aattaactta tgctcaagtg agagcaatgc ttgcgttatc tgataatgct 960

attttcaaag gggttcgtta tttaggcgaa gataaaaaaa cagtagagag caaaactacg 1020

ttgatagaaa tgaagtttta tcatcaaatc cgcaaaacat taggcagtgc agaattaaaa 1080

aaggaatgga atgagttaaa aggcaattcc gatttattag atgagattgg cacggcattt 1140

tcgttgtata aaacggatga tgatatttgc cgttatttag agggaaaact accagaaagg 1200

gtattaaatg cgttattgga aaatttaaat ttcgataaat ttattcaact ttcacttaaa 1260

gccttacacc aaattttacc attgatgctg caagggcaac gttatgatga ggcggtttct 1320

gcgatttatg gtgatcatta tggtaaaaaa tcgacagaaa caacccgctt gttgccgact 1380

attcctgccg atgaaatccg aaatcctgtg gtattacgca ccctgaccca agcccgtaaa 1440

gtgatcaatg cggtggtgcg gttatatggt tcgcctgccc gtattcatat tgaaacagcg 1500

agagaagtcg gcaaatctta ccaagatcgt aaaaaacttg aaaaacagca agaagataat 1560

cgtaagcaac gtgaaagtgc ggtcaaaaaa tttaaagaaa tgtttccgca ttttgtgggg 1620

gagccgaaag gtaaagatat tttaaaaatg cgattgtatg agttacaaca agcgaaatgt 1680

ttatattctg gaaaatcttt agaacttcat cgtttgcttg agaaggggta tgtagaagtg 1740

gatcacgctt tgccattttc tcgcacgtgg gatgatagct ttaataataa agtactggtg 1800

cttgccaacg agaaccaaaa taaaggcaat ttaacgcctt atgaatggtt agatggtaaa 1860

aataacagtg agcgttggca acattttgtt gtacgagtac aaaccagcgg tttctcttat 1920

gctaaaaaac aacgcatttt gaaccataaa ttggatgaaa aagggtttat cgaacgtaat 1980

ttaaacgata ctcgctatgt agctcgtttc ttatgtaact ttattgccga taatatgttg 2040

ttggttggta aaggcaagcg aaacgtgttt gcttcaaacg ggcaaatcac ggcgttattg 2100

cggcatcgtt ggggcttaca aaaagtgcgt gaacagaatg atcgccacca cgcactggac 2160

gcggttgtgg tggcttgctc tactgtggca atgcaacaaa aaatcactcg atttgtgaga 2220

tataacgaag gaaatgtctt tagcggtgaa cgtatcgatc gtgaaactgg cgagattatt 2280

ccattacatt ttccaagccc ttgggctttt ttcaaagaga atgtggaaat tcgcattttt 2340

agtgaaaatc caaaattgga attagaaaat cgcctgcctg attatccgca atataatcac 2400

gaatgggtgc aaccattgtt tgtttcgaga atgccaaccc gaaaaatgac agggcaaggg 2460

catatggaaa cggtaaaatc cgcaaaacga ttaaatgaag gtttaagtgt gttaaaagtc 2520

cctttaacac aacttaaatt gagtgattta gaacgaatgg ttaatcgtga tcgtgaaatt 2580

gcattgtatg aatccttaaa agcacgttta gagcaatttg gtaacgaccc agccaaagcc 2640

tttgccgaac cattctataa aaagggtggg gcattagtca aagcagtccg attggaacaa 2700

acacaaaaat cgggggtatt agtacgtgat ggtaacggtg ttgcggataa tgcttcaatg 2760

gtacgggttg atgtttttac taaaggtgga aaatatttct tagtgccgat ttatacttgg 2820

caggtagcga aagggatttt accgaatagg gctgcgacac aaggtaaaga tgaaaatgat 2880

tgggatatta tggatgaaat ggctactttc caattttctc tatgtcaaaa tgatctaatt 2940

aaattagtta ccaaaaagaa aacaatcttt ggatatttta atggattaaa tagagctact 3000

agcaatataa atattaaaga gcatgatcta gataagtcta aagggaaatt aggtatttac 3060

ttagaagttg gtgtaaaact agctatttcc cttgaaaagt accaagtcga cgaactcggc 3120

aaaaatatcc gtccttgtcg tccgactaaa cgacagcacg tgcgttaa 3168